Europäisches Patentamt

**European Patent Office**

Office européen des brevets

(11) Numéro de publication : **0 129 451
B1**

(12) **FASCICULE DE BREVET EUROPÉEN**

(45) Date de publication du fascicule du brevet :
02.12.87

(51) Int. Cl.⁴ : **A 61 B   6/14**, G 01 T   1/20

(21) Numéro de dépôt : **84400886.2**

(22) Date de dépôt : **02.05.84**

(54) **Appareil permettant d'obtenir une image radiologique dentaire, et plus particulièrement capteur intra-buccal mis en oeuvre dans un tel appareil.**

(30) Priorité : **16.06.83 FR 8310277**

(43) Date de publication de la demande :
**27.12.84 Bulletin 84/52**

(45) Mention de la délivrance du brevet :
**02.12.87 Bulletin 87/49**

(84) Etats contractants désignés :
**AT BE CH DE FR GB IT LI LU NL SE**

(56) Documents cités :
**EP-A- 0 000 079
DE-A- 2 527 750
FR-A- 2 108 385
FR-A- 2 378 496
FR-A- 2 476 949
US-A- 3 051 166
US-A- 4 160 997
US-A- 4 304 998
PATENTS ABSTRACTS OF JAPAN, vol. 7, no. 6 (P-167)[1151], 11 janvier 1983;
PHYSICS IN MEDICINE & BIOLOGY, vol. 23, no. 5, septembre 1978, pages 993-997, Londres, GB; LO I YIN et al.: "The lixiscope: A pocket-size X-ray imaging system"**

(73) Titulaire : **Mouyen, Francis
12, Avenue d'Occitanie
F-31520 Ramonville-Saint-Agne (FR)**

(72) Inventeur : **Mouyen, Francis
12, Avenue d'Occitanie
F-31520 Ramonville-Saint-Agne (FR)**

(74) Mandataire : **Morelle, Guy Georges Alain
Cabinet SCOPI 1, Avenue de Rangueil
F-31400 Toulouse (FR)**

## Description

L'invention a trait aux techniques de radiologie dentaire et concerne plus particulièrement un appareil permettant d'obtenir une image radiologique dentaire sur le moniteur d'une chaîne de visualisation.

L'évolution de l'électronique observée ces dernières années a permis de faire progresser notablement les techniques d'examens radiologiques des organes du corps humain. Cette évolution vise plus particulièrement à réduire, pour le patient et pour le manipulateur, les doses d'exposition aux rayons X, tout en améliorant la qualité d'image de la cible radiographiée. Les procédés et les dispositifs décrits et représentés dans les brevets français n° 2.333.404, n° 2.378.496, n° 2.415.938, n° 2.495.429, n° 2.476.949, n° 2.477.626, n° 2.479.636, n° 2.185.667, n° 2.247.749 et n° 2.310.059, démontrent bien cet état de fait.

Les techniques d'exploration dentaire en sont, quant à elles, restées au stade traditionnel de la radiographie qui consiste à intercaler la dent à examiner entre une source extra-buccale de rayons X et un film radiographique intra-buccal, sensible aux rayons X traversant la dent irradiée. Les formes de l'image obtenue sur ce film radiographique correspondent aux ombres portées par les constituants plus ou moins opaques aux rayons X de la dent examinée. Bien que cette technique de radiographie dentaire soit la plus usitée à l'heure actuelle, elle présente toutefois l'inconvénient de limiter le nombre des clichés compte tenu des doses de rayons X qu'ils exigent.

Il est utile de préciser que les nouvelles techniques de radiologie ont plus particulièrement porté sur la réalisation intrinsèque du capteur du faisceau de rayons X émergeant de la cible irradiée afin, comme on l'a rappelé ci-dessus, de réduire les temps d'exposition aux rayons X tout en améliorant la qualité d'image de la cible radiographiée. De plus, l'image est obtenue en temps réel, évitant ainsi les manipulations de développement du film de la radiographie dentaire traditionnelle.

Or, l'application d'un appareil utilisant ces nouvelles techniques à la radiologie dentaire se heurte à de sérieux problèmes car le capteur de faisceau de rayons X doit respecter certaines dimensions maximales pour permettre une introduction facile en bouche. La difficulté majeure de réalisation d'un tel appareil, et plus particulièrement du type de celui permettant d'obtenir une image radiologique dentaire sur un moniteur d'une chaîne de visualisation, réside donc dans le fait de donner au capteur de faisceau de rayons X de la dent irradiée la plus faible épaisseur possible pour qu'il trouve place dans la cavité buccale derrière toutes les dents qui sont susceptibles d'être examinées.

Une réalisation d'un tel appareil a déjà été décrite et représentée dans le brevet américain n° 4.160.997 (Schwartz) dans lequel il est proposé un capteur utilisant notamment un dispositif à transfert de charges devant lequel est disposé un écran destiné à transformer les rayons X émergeant de la dent irradiée en rayons de longueur d'onde acceptable par ledit dispositif. Le dispositif à transfert de charges ayant un rendement optimum dans le spectre visible, le susdit écran comporte en entrée un scintillateur qui, sous l'impact des rayons X, transforme une grande partie de ces derniers en rayons de longueur d'onde visible. Il est certain que l'utilisation d'un dispositif à transfert de charges peut, par ses faibles dimensions, remplir les conditions requises pour la réalisation d'un capteur intra-buccal de faible encombrement et permettant de réduire notablement les doses d'exposition aux rayons X tout en offrant une qualité supérieure d'image. Les caractéristiques et avantages techniques d'un dispositif à transfert de charges sont connus par l'homme de l'art et ne seront pas rappelés dans le présent mémoire.

La réalisation du capteur intra-buccal, décrite dans ce brevet américain, présente de nombreux inconvénients et en particulier l'inconvénient majeur de ne pas pouvoir remplir sa fonction essentielle qui consiste à enregistrer le faisceau de rayons X émergeant d'une dent irradiée et à fournir en sortie des informations susceptibles d'être analysées par une unité de traitement électronique, ceci afin de faire apparaître, sur le moniteur d'une chaîne de visualisation, l'image de la susdite dent. En effet, pour comprendre que ce capteur intra-buccal n'est pas fonctionnel, il est utile de rappeler que les dispositifs à transfert de charges présentent les particularités suivantes :

les dimensions de leur face sensible ne sont pas suffisantes pour capter tous les rayons X d'un faisceau émergeant d'une dent irradiée et Schwartz propose d'utiliser, dans son capteur buccal, un écran assurant une transmission linéaire ;

leur face sensible se détériore sous l'impact de rayons X d'énergie supérieure à 1 KeV et l'écran de Schwartz n'assure pas une protection suffisante de cette face sensible ;

l'unité de traitement électronique des informations électriques en sortie du dispositif à transfert de charges ne doit pas être éloignée de ce dernier de plus de 20 cm (distance limite au-delà de laquelle le signal de sortie est trop faible pour être traité) et l'unité de traitement électronique des informations en sortie du capteur intra-buccal de Schwartz est extra-buccale et reliée à ce dernier par un câble d'une longueur de plus de 20 cm.

Faisant le bilan de cet état de fait, le demandeur a mené des recherches qui ont abouti à la réalisation d'un appareil permettant d'obtenir une image radiologique dentaire sur un moniteur d'une chaîne de visualisation et d'obvier aux inconvénients précités, pour offrir un appareil fonctionnel aux performances incontestables dans la qualité de reproduction de l'image dentaire et dans la réduction de la dose d'exposition aux rayons X.

Selon l'invention, l'appareil comportant :

une source extra-buccale de rayons X,

un capteur intra-buccal de rayons X ayant traversé la dent irradiée et disposé coaxialement au faisceau de rayons X émergeant de cette dernière,

et une unité de traitement électronique extra-buccale reliée au capteur, ledit capteur étant formé par l'association d'un dispositif à transfert de charges et d'un écran qui, interposé entre ledit dispositif et la dent irradiée, est pourvu en entrée d'un scintillateur transformant les rayons X ayant traversé la dent en rayons visibles, ledit écran étant constitué d'une épaisseur de matière translucide perméable aux rayons visibles et garnie de particules destinées à s'opposer au passage des rayons X traversant le scintillateur et non transformés par ce dernier en rayons visibles, est remarquable en ce que :

d'une part, les diamètres des parties élémentaires des couples d'éléments en regard ont des valeurs progressives dans le sens de la transmission de l'image dentaire-scintillateur (6), écran (5), dispositif à transfert de charges (4), à savoir le premier couple d'éléments en regard constitué par les cristaux du scintillateur (6) et les faces d'entrée des fibres optiques en entrée de l'écran (5) et le deuxième couple d'éléments en regard constitué par les faces de sortie des fibres optiques en sortie de l'écran (5) et les points élémentaires de lumière du dispositif à transfert de charges (4), afin d'optimiser le pouvoir de résolution des points élémentaires de lumière du dispositif à transfert de charges (4) par diminution des effets de moirage,

d'autre part, le susdit écran est formé par des fibres optiques réductrices garnies de particules d'oxydes métalliques destinées à absorber l'énergie des rayons X non transformés par le scintillateur,

et enfin, le susdit capteur intra-buccal comporte une micro-électronique de pilotage du dispositif à transfert de charges et d'amplification du signal de sortie de ce dernier.

On comprend que la réalisation du capteur intra-buccal de l'invention telle que décrite ci-dessus ne se limite pas à une simple association d'un scintillateur, d'un écran et d'un dispositif à transfert de charges, qui ne pourrait pas fonctionner, mais à une réalisation et à une association originales de ces trois constituants formant une chaîne optique et qui ont été étudiées pour une application à la radiologie dentaire.

Ainsi, le fait de donner une croissance progressive aux diamètres des parties élémentaires en regard des différents constituants de la chaîne optique, dans le sens de la transmission de l'image dentaire, permet notamment d'éviter un chevauchement desdites parties élémentaires « émettrices » sur les parties élémentaires « réceptrices », chevauchement qui provoque les effets de moirage. En effet, l'objet recherché est d'obtenir un grand nombre de cristaux du scintillateur en regard d'une fibre optique en entrée de l'écran qui soit équilibré avec un grand nombre de fibres optiques en sortie de l'écran en regard de chaque partie élémentaire de lumière de la face sensible du dispositif à transfert de charges. Cet équilibre est notamment obtenu en prenant pour les cristaux du scintillateur des dimensions de l'ordre de 3 à 4 $\mu$, pour les fibres optiques en entrée de l'écran des dimensions de l'ordre de 9 à 14 $\mu$ et, en sortie de l'écran, de l'ordre de 4,5 à 7 $\mu$, et pour les points élémentaires de lumière du dispositif à transfert de charges des dimensions de l'ordre de 24 à 34 $\mu$.

De plus, l'utilisation de fibres optiques réductrices, de coefficient réducteur d'environ 2, permet d'avoir, entre l'entrée et la sortie de l'écran, une transmission réduite de l'image dentaire qui, d'une part, augmente le nombre de fibres optiques en regard de chaque point élémentaire de lumière du dispositif à transfert de charges, d'autre part, adapte les dimensions de la face sensible du dispositif à transfert de charges à celles de la surface d'entrée de l'écran (imposées par les dimensions du faisceau de rayons X émergeant d'une dent irradiée et correspondant aux dimensions de cette dernière), et enfin multiplie par 4 le rapport de l'intensité lumineuse sur la surface, et par conséquent autorise dans le même rapport (4) une réduction de la dose d'exposition de rayons X.

Les fibres optiques, façonnées de préférence dans du verre stabilisé à faible coefficient de brunissement pour une parfaite transparence au passage des rayons visibles, sont avantageusement garnies de particules d'oxydes métalliques dont la masse atomique est supérieure de 50 % à la masse atomique du silicium dans lequel sont étirées les fibres optiques afin d'absorber, dans de bonnes conditions, l'énergie des rayons X non transformés par le scintillateur. La présence de ces particules d'oxydes métalliques assure une protection du dispositif à transfert de charges, en établissant une barrière au passage des rayons X non acceptés par ledit dispositif. Ces particules d'oxydes métalliques présentent également, selon une caractéristique particulièrement avantageuse de l'invention, une structure chimique tétravalente et un point de fusion supérieur à 1 500 °C afin de permettre l'étirement des fibres optiques réductrices contenant ces particules jusqu'à des diamètres de l'ordre de 4,5 à 7 $\mu$, correspondant à ceux des fibres optiques en sortie de l'écran.

Enfin, le demandeur a également imaginé d'incorporer, dans le capteur intra-buccal, une micro-électronique de pilotage du dispositif à transfert de charges et d'amplification du signal de sortie de ce dernier. Cette micro-électronique apporte ainsi une solution au problème de la transmission, à une distance supérieure à 20 cm du dispositif à transfert de charges, des informations électriques en sortie de ce dernier. Pour y parvenir, il assure une amplification desdites informations qui permet de transmettre, à l'unité de traitement électronique de la chaîne de visualisation, un signal électrique exploitable par celle-ci. Cependant, l'installation de cette micro-électronique dans le capteur intra-buccal pose quelques difficultés car, par sa présence, elle peut augmenter l'encombrement du dispositif à transfert de charges et également provoquer une élévation de température de ses composants, entraînant un échauffement au voisinage du dispositif, échauffement qui aurait pour conséquence immédiate d'augmenter le bruit de fond de ce dernier. Pour surmonter les difficultés d'encombrement de cette micro-électronique, le demandeur propose, selon une caractéristique particulièrement avantageuse de l'invention, d'une part de retirer la puce qui constitue la partie fonctionnelle du dispositif à transfert de charges et, d'autre part, de coller sa face sensible en regard des rayons visibles sur les fibres optiques en sortie de l'écran, et sa face opposée sur un support en céramique sur lequel la micro-électronique est disposée selon la technique de multicouches pour des raisons de miniaturisation. On comprend ainsi que l'espace occupé par les différents constituants de la chaîne optique dans l'axe de propagation de l'image est réduit au maximum. A ce sujet, il est important de préciser que, compte tenu de l'épaisseur totale du capteur intra-buccal, qui doit être proche de 17 mm pour permettre une introduction facile dans la cavité buccale, de l'épaisseur du scintillateur qui sera inférieure à 100 μ pour limiter le problème de diffusion optique, et de l'épaisseur de l'écran qui doit respecter une dimension limite minimale pour assurer une réduction suffisante (rapport 2) permettant d'adapter la surface de la dent irradiée à celle de la puce, seule l'épaisseur du dispositif à transfert de charges pouvait faire l'objet d'une miniaturisation.

Le problème d'échauffement de la micro-électronique est, quant à lui, résolu en dissociant certains composants susceptibles d'élévation de température et notamment les composants assurant une transformation des tensions d'alimentation, et en les disposant sur des prolongements latéraux, perpendiculaires au support-céramique. Cette caractéristique de l'invention offre le grand avantage d'éloigner de la puce les composants chauffants et ainsi de diminuer le bruit de fond de cette dernière. Selon une caractéristique particulièrement avantageuse de l'invention, permettant également de limiter l'élévation de température des composants électroniques chauffants, l'alimentation en courant électrique de la microélectronique de pilotage et d'amplification du signal de sortie est synchronisée avec celle du générateur de la source de rayons X pour une durée nécessaire à l'obtention d'une seule image sur le moniteur de la chaîne de visualisation sur lequel elle sera maintenue pour les travaux ultérieurs d'exploration. Cette limite de durée d'alimentation en énergie, expérimentalement déterminée à quelques centièmes de seconde, offre le double avantage de restreindre considérablement la dose d'exposition aux rayons X et l'élévation de température des composants électroniques chauffants, ceci afin de pouvoir introduire la micro-électronique dans le capteur intra-buccal dans le souci d'une miniaturisation de ce dernier.

Selon une autre réalisation préférentielle de l'invention, le scintillateur est appliqué sur les fibres optiques en entrée du susdit écran par un dépôt de couches de cristaux de granulométrie constante de l'ordre de 3 à 4 μ, dépôt dont l'épaisseur totale est inférieure à 100 μ afin d'éviter les phénomènes de diffusion optique à l'intérieur dudit scintillateur. La longueur d'onde d'émission de ces cristaux est comprise entre 500 et 800 nm (nanomètres), correspondant à la plage de réponse optimale des points élémentaires de lumière de la puce. Ce mode de réalisation du scintillateur a été conçu afin d'améliorer la qualité d'image reçue.

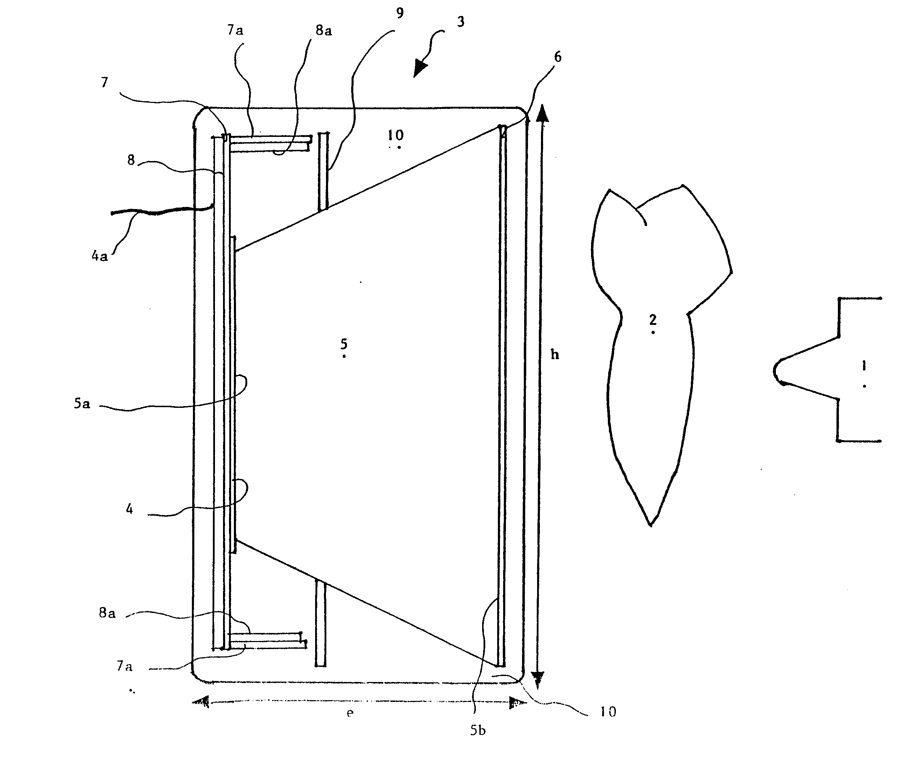

D'autres caractéristiques et d'autres avantages de la présente invention apparaîtront plus clairement à la lecture de la description ci-après, donnant, à titre d'exemple non limitatif et en regard du dessin annexé, un mode de réalisation d'un appareil permettant d'obtenir une image radiologique dentaire, selon les concepts fondamentaux de l'invention.

La figure portée par ce dessin est une vue en coupe latérale schématique d'un tel appareil.

Cet appareil comporte :

un générateur de rayons X extra-buccal 1, destiné à irradier une dent 2 logée par exemple dans la mâchoire inférieure d'une bouche,

un capteur intra-buccal 3 des rayons X ayant traversé la dent 2 derrière laquelle il est positionné,

et une unité de traitement électronique extrabuccale, non représentée, qui enregistre les informations électriques en sortie du capteur intrabuccal 3 auquel elle est reliée par un câble souple 4a, afin de faire apparaître l'image de la dent 2 sur le moniteur d'une chaîne de visualisation non représentée.

L'invention repose essentiellement sur la conception du capteur intra-buccal 3 qui, d'une part, doit pouvoir enregistrer l'entière image de la dent 2 irradiée et, d'autre part, doit être suffisamment miniaturisé pour pouvoir être placé aisément derrière celle-ci dans la cavité buccale. Afin de mieux comprendre la démarche opérée par l'inventeur destinée à donner au capteur intrabuccal les dimensions les plus petites possibles tout en restant fonctionnel, le dessin schématique du capteur 3 a été représenté à l'échelle 5.

Ce capteur 3 adoptant, avec son matériau d'enrobage, la forme d'un parallélépipède rectangle, d'une hauteur (h) d'environ 30 mm, d'une largeur (l) d'environ 20 mm et une épaisseur maximale (e) de 17 mm, pour concilier une introduction facile en bouche avec un enregistrement total du faisceau de rayons X émergeant d'une dent.

Conformément à l'invention, le capteur intrabuccal 3 est constitué de la puce 4 d'un dispositif à transfert de charges collée sur la face 5a du plan tronqué d'un écran 5 adoptant la configuration générale d'une pyramide tronquée dont le plan de base 5b est recouvert d'un scintillateur 6 qui transforme les rayons X émergeant de la dent 2 irradiée en rayons du spectre visible qui, comme nous l'avons rappelé dans la description, seront enregistrés dans de meilleures conditions par la puce 4, notamment en déposant sur le plan de base 5b des cristaux émettant dans une longueur d'onde de l'ordre de 500 à 800 nm qui correspond à la plage de réponse optimale de la puce 4.

L'écran 5, dont les dimensions du plan de base 5b en entrée sont telles qu'elles définissent une surface suffisamment grande pour recevoir tous les rayons X émergeant de la dent 2, et dont celles du plan tronqué 5a en sortie sont telles qu'elles définissent une surface suffisamment petite pour être recouverte par la puce 4, est formé de fibres optiques réductrices, d'un rapport voisin de 2, façonnées dans un verre stabilisé garni de particules d'oxydes métalliques.

Outre le fait de réduire l'image dentaire entre l'entrée 5b et la sortie 5a de l'écran, les fibres optiques permettent de conduire point par point l'image dentaire entre le scintillateur 6 et la puce 4 et de diminuer la dose d'exposition aux rayons X car le pouvoir réducteur des fibres augmente l'intensité lumineuse, dans un rapport proportionnel au carré du coefficient réducteur des fibres.

Les particules d'oxydes métalliques garnissant les fibres optiques sont choisies de manière à ce que leur masse atomique soit supérieure de 50 % à celle du silicium afin qu'elles puissent absorber une grande partie de l'énergie des rayons X non transformés par le scintillateur 6, et assurer ainsi un rôle protecteur pour la puce 4. En outre, la structure chimique de l'oxyde métallique choisi doit posséder une couche électronique périphérique à quatre valences et son point de fusion doit être supérieur à 1 500 °C afin d'offrir respectivement une comptabilité chimique et physique avec le silicium, pour donner une plage de viscosité permettant d'étirer les fibres optiques jusqu'à des diamètres de l'ordre de 4,5 à 7 µ.

Ainsi, les rayons X traversant la dent 2 vont, au contact des cristaux du scintillateur 6, émettre des rayons lumineux représentant une image de la dent 2 dans le spectre visible qui sera conduite et rétrécie point par point, par les fibres optiques de l'écran 5, jusqu'à l'entrée de la puce 4. Selon l'invention, cette dernière a été retirée de son boîtier afin d'être reportée sur un support-céramique 7 de faible épaisseur, collé sur la face de la puce 4 opposée à celle en regard de l'écran 5. Ce support comporte une micro-électronique 8 destinée à piloter la puce 4 et à amplifier le signal de sortie de cette dernière afin de pouvoir transmettre, à l'extrémité du câble 4a, un signal électrique exploitable par l'unité de traitement électronique de la chaîne de visualisation.

Afin d'éviter le problème d'échauffement au voisinage de la puce 4 provoqué par l'élévation de température de certains composants électroniques qui ont tendance à chauffer, notamment les composants qui assurent une transformation de tension, ceux-ci, référencés 8a dans leur ensemble, sont dissociés de la micro-électronique 8 et disposés sur des prolongements latéraux et perpendiculaires 7a au support-céramique 7.

Selon une caractéristique particulièrement avantageuse de l'invention, une ceinture de plomb 9, dont le plan est parallèle au plan de base 5b de la pyramide tronquée 5, est disposée autour des faces latérales de cette dernière, sur une hauteur, par rapport au plan de base 5b, égale aux deux-tiers de la hauteur totale de la pyramide. Cette ceinture de plomb 9 assure, pour les constituants disposés en aval (puce 4, micro-électronique 8), une protection aux rayons X non transformés par le scintillateur 6 et qui ont une trajectoire linéaire à travers l'épaisseur de l'écran 5 situé en amont de la ceinture 9. Cette épaisseur, représentant les deux-tiers de l'épaisseur totale de l'écran 5 qui correspond à la hauteur de la pyramide, a été déterminée expérimentalement comme une épaisseur minimale qui assure une absorption suffisante des rayons X.

Avantageusement, toutes les surfaces extérieures des constituants décrits ci-dessus (puce 4, écran 5, scintillateur 6, support-céramique 7, micro-électronique 8 et ceinture de plomb 9) sont noyées dans un matériau d'enrobage 10, de préférence une résine polyuréthane de couleur noire qui assure à la fois :

un passage sélectif à certaines longueurs d'ondes, notamment en laissant passer les rayons X et en s'opposant au passage des rayons visibles extérieurs au capteur 3 afin de ne pas perturber la réception des rayons X par le scintillateur 6,

un coefficient de conductibilité thermique faible pour limiter l'échauffement des composants électroniques au voisinage de la puce 4,

un rôle d'isolant électrique,

et une protection mécanique du capteur contre les chocs.

Selon une autre caractéristique de réalisation préférée de l'invention, le capteur 3 sera logé dans une enveloppe aseptique jetable bio-compatible avec la cavité buccale et non représentée sur le dessin.

Afin d'installer le capteur 3 en position de réception du faisceau de rayons X émergeant d'une dent irradiée à l'intérieur d'une bouche, le capteur 3 sera fixé, de manière amovible et articulée, à l'extrémité d'un bras (non représenté) dont l'autre extrémité est libre pour être calée entre deux dents antagonistes.

On comprendra que les réalisations et pratiques spécifiques décrites ci-dessus l'ont été en vue d'une divulgation plutôt que d'une limitation et que de nombreuses modifications, combinaisons et substitutions peuvent être effectuées par les hommes de l'art sans s'éloigner de la portée de l'invention.

**Revendications**

1. Appareil permettant d'obtenir une image radiologique dentaire sur un moniteur d'une chaîne de visualisation et comportant :

une source extra-buccale (1) de rayons X,

un capteur intra-buccal (3) de rayons X ayant traversé la dent irradiée (2) et disposé coaxialement au faisceau de rayons X émergeant de cette dernière,

et une unité de traitement électronique extra-buccale reliée au capteur (3), ledit capteur étant formé par l'association d'un dispositif à transfert de charges (4) et d'un écran (5) qui, interposé entre ledit dispositif et la dent (2) irradiée, est pourvu en entrée d'un scintillateur (6) transformant les rayons X ayant traversé la dent (2) en rayons visibles, ledit écran (5) étant constitué d'une épaisseur de matière translucide perméable aux rayons visibles et garnie de particules destinées à s'opposer au passage des rayons X traversant le scintillateur (6) et non transformés par ce dernier en rayons visibles, caractérisé par le fait que :

d'une part, les diamètres des parties élémentaires des couples d'éléments en regard ont des valeurs progressives dans le sens de la transmission de l'image dentaire-scintillateur (6), écran (5), dispositif à transfert de charges (4), à savoir le premier couple d'éléments en regard constitué par les cristaux du scintillateur (6) et les faces d'entrée des fibres optiques en entrée de l'écran (5) et le deuxième couple d'éléments en regard constitué par les faces de sortie des fibres optiques en sortie de l'écran (5) et les points élémentaires de lumière du dispositif à transfert de charges (4), afin d'optimiser le pouvoir de résolution des points élémentaires de lumière du dispositif à transfert de charges (4) par diminution des effets de moirage,

d'autre part, le susdit écran (5) est formé par des fibres optiques réductrices garnies de particules d'oxydes métalliques destinées à absorber l'énergie des rayons X non transformés par le scintillateur (6),

et enfin, que le susdit capteur intra-buccal (3) comporte une micro-électronique (8) de pilotage du dispositif à transfert de charges et d'amplification du signal de sortie de ce dernier.

2. Appareil selon la revendication 1, caractérisé par le fait que les cristaux du scintillateur (6) ont une dimension de l'ordre de 3 à 4 μ, les fibres optiques ont une dimension, en entrée de l'écran (5) de l'ordre de 9 à 14 μ et en sortie de l'écran (5) de l'ordre de 4,5 à 7 μ, et les points élémentaires de lumière du dispositif à transfert de charges (4) ont une dimension de l'ordre de 24 à 34 μ.

3. Appareil selon la revendication 1, caractérisé par le fait que les fibres optiques de l'écran (5) sont façonnées dans du verre stabilisé à faible coefficient de brunissement.

4. Appareil selon la revendication 1, caractérisé par le fait que la puce (4) constituant la partie fonctionnelle du dispositif à transfert de charges est retirée de son boîtier et, par le fait que sa face sensible réceptrice des rayons visibles est collée sur les fibres optiques en sortie (5a) de l'écran (5) alors que sa face opposée est collée à un support-céramique (7) de la susdite micro-électronique (8) de pilotage de la puce et d'amplification du signal de sortie de cette dernière, cette micro-électronique (8) étant disposée selon la technique multi-couches.

5. Appareil selon les revendications 1 et 2, caractérisé par le fait que le susdit scintillateur (6) est appliqué sur les fibres optiques en entrée (5b) du susdit écran (5) par un dépôt de couches de cristaux de granulométrie constante de l'ordre de 3 à 4 μ, dépôt dont l'épaisseur totale est inférieure à 100 μ afin d'éviter les phénomènes de diffusion optique, et dont la longueur d'onde d'émission est comprise entre 500 et 800 nm (nanomètres) correspondant à la plage de réponse optimale du dispositif à transfert de charges.

6. Appareil selon les revendications 1, 2 et 3, caractérisé par le fait que les particules d'oxydes métalliques garnissant les fibres optiques du susdit écran (5) ont, d'une part, une masse atomique supérieure de plus de 50 % à la masse atomique du silicium constituant le verre stabilisé dans lequel sont façonnées les fibres optiques, afin d'augmenter le pouvoir d'absorption de l'écran agissant sur les rayons X non transformés en rayons visibles par le scintillateur (6) et, d'autre part, une structure chimique tétravalente et un point de fusion supérieur à 1 500 °C, afin de faciliter l'étirement des fibres optiques réductrices pour l'obtention de diamètres de l'ordre de quelques μ.

7. Appareil selon les revendications 1 et 4, caractérisé par le fait que l'alimentation en courant électrique de la micro-électronique (8) de pilotage de la puce (4) du dispositif à transfert de charges et d'amplification du signal de sortie de ladite puce est synchronisée avec celle du générateur (1) de la source de rayons X et a une durée minimum nécessaire à l'obtention d'une seule image sur le moniteur de la chaîne de visualisation sur lequel elle sera maintenue pour les travaux d'exploration ultérieurs.

8. Appareil selon les revendications 1, 2, 3, 5 et 6, prises ensemble, caractérisé par le fait que :

d'une part, l'écran (5), qui est formé par l'ensemble des fibres optiques réductrices, adopte la forme générale d'une pyramide tronquée selon un plan (5a) parallèle au plan de base (5b),

d'autre part, le scintillateur (6) est déposé sur le plan de base (5b) de la pyramide et la face comportant la partie sensible de la puce (4) est collée sur le plan tronqué (5a) de ladite pyramide (5),

et enfin, une ceinture de plomb (9), dont le plan est parallèle au plan de base (5b) de la pyramide, est disposée autour des faces latérales de cette dernière, sur une hauteur, par rapport au plan de base où se trouve le scintillateur, égale aux deux-tiers de la hauteur totale de la pyramide.

9. Appareil selon les revendications 1, 4 et 7, caractérisé par le fait que certains composants (8a) de la susdite micro-électronique (8) de pilotage de la puce (4) et d'amplification du signal de sortie de cette dernière, susceptibles de provoquer une élévation de température au voisinage de ladite puce, sont disposés sur des prolongements latéraux, perpendiculaires (7a) au support-céramique (7) des autres composants électroniques adossé à la susdite puce (4).

10. Appareil selon l'ensemble des revendications 1 à 9, caractérisé par le fait que toutes les surfaces extérieures des constituants du capteur intra-buccal (3) du faisceau de rayons X émergeant de la dent irradiée (2) sont noyées dans un matériau d'enrobage (10) qui assure à la fois :

un passage sélectif à certaines longueurs d'onde de rayons, notamment en laissant passer les rayons X et en s'opposant au passage des rayons visibles extérieurs au capteur (3),

un coefficient de conductibilité thermique faible afin de limiter l'échauffement de la puce (4) du dispositif à transfert de charges,

un rôle d'isolant électrique,

et une protection mécanique du capteur (3) contre les chocs.

11. Appareil selon l'ensemble des revendications 1 à 10, caractérisé par le fait que les dimensions des différents constituants du capteur intra-buccal (3) du faisceau de rayons X émergeant de la dent irradiée (2) sont déterminées de manière à ce que :

celles de l'ensemble du capteur intra-buccal (3) assurent une manipulation facile d'introduction et de retrait du capteur en bouche,

celles du plan de base (5b) de la pyramide tronquée dudit écran (5), sur lequel sont déposés les cristaux du scintillateur (6), permettent de recevoir, en entrée de l'écran, l'image entière du faisceau de rayons X émergeant d'une dent irradiée (2) au moins,

celles du plan tronqué (5a) de la pyramide du susdit écran (5), sur lequel est collée à la face sensible de la puce (4) d'un dispositif à transfert de charges, permettent d'obtenir, en sortie de l'écran (5), une image dentaire suffisamment réduite pour être enregistrée par la face sensible de la susdite puce (4),

et celles de la hauteur de la pyramide tronquée du susdit écran (5), correspondant à la longueur des fibres optiques et plus précisément à l'épaisseur de verre garnie de particules d'oxydes métalliques, permettent une absorption suffisante de l'énergie des rayons X non transformés par le scintillateur (6), pour protéger la face sensible de la susdite puce.

12. Appareil selon la revendication 10, caractérisé par le fait que le susdit matériau d'enrobage (9) des surfaces extérieures des différents constituants du susdit capteur intra-buccal (3) est constitué par de la résine polyuréthane de couleur noire.

13. Appareil selon la revendication 11, caractérisé par le fait que les dimensions de l'ensemble du capteur intra-buccal (3) sont définies par celles d'un rectangle en entrée correspondant au plan de base de la pyramide tronquée et limitées à 30 mm de hauteur (h) sur 20 mm de largeur (l) et par celles de l'épaisseur totale (e) correspondant à la somme des épaisseurs du scintillateur (6), de l'écran (5), de la puce (4), de la micro-électronique (8), du matériau d'enrobage, épaisseur (e) limitée à 17 mm.

14. Appareil selon l'ensemble des revendications 1 à 13, caractérisé par le fait que le susdit capteur intra-buccal (3) est maintenu dans la cavité buccale au moyen d'un bras articulé et amovible.

15. Appareil selon l'ensemble des revendications 1 à 14, caractérisé par le fait que le susdit capteur intra-buccal (3) est logé dans une enveloppe aseptique jetable.

**Claims**

1. An apparatus for providing a dental radiological image on the monitor of a display system, said apparatus comprising :

an extraoral X-ray source (1) ;

an intraoral sensor (3) for the X-rays passing through a radiated tooth (2), said sensor being coaxial to the X-ray beam emerging from said tooth ;

and an extraoral electronic data processing unit connected to said sensor (3), said sensor comprising a charge-coupled device (4) and a screen (5), said screen being located between the charge-coupled device and the radiated tooth (2), and being provided on entry with a scintillator (6) which converts the X-rays which have passed through the tooth (2) into visible radiation, said screen (5) being made of a layer of translucid material permeable to visible radiation, said translucid material being loaded with particles intended for preventing the passage of X-rays passing through said scintillator (6) and which have not been converted into visible radiation by the scintillator, characterized in that

the diameters of the elemental parts of opposite pairs of elements are progressively increased along the direction of the transmission of the dental image-scintillator (6), screen (5), and charge-coupled device (4), the first pair of opposite elements being constituted by the crystals of said scintillator (6) and the admission sides of the optical fibers on entry of said screen (5), and the second pair of opposite elements being constituted by the output side of the optical fibers on issue of said screen (5) and the picture elements of the charge-coupled device (4), for an optimization of the capacity of resolution of picture elements in the charge-coupled device (4) upon reducing the double moiré effect,

said screen (5) comprises reducing optical fibers loaded with metallic oxide particles intended to absorb the X-ray energy which has not been converted by said scintillator (6),

and said intraoral sensor (3) comprises a microelectronic means (8) for monitoring the charge-coupled device and for amplifying the output signal of the charge-coupled device.

2. An apparatus as set forth in Claim 1, characterized in that the crystals of the scintillator (6) have a 3-4 $\mu$ diameter, said reducing optical fibers have a 9-14 $\mu$ diameter on entry of the screen (5) and a 4.5-7 $\mu$ diameter on issue of said screen (5), and said picture elements of the charge-coupled device (4) have a 24-34 μ diameter.

3. An apparatus as set forth in Claim 1, characterized in that the optical fibers of said screen (5) are made of stabilized glass with a low darkening coefficient.

4. An apparatus as set forth in Claim 1, characterized in that said charge-coupled device comprises a chip (4) constituting its functional part, said chip being withdrawn from its casing and having its sensible face stuck on the optical fibers on issue (5a) of said screen (5) and having its opposite face stuck on a ceramic substrate (7), said ceramic substrate further supporting said microelectronic means (8) for monitoring the chip and for amplifying the output signal of the chip, said microelectronic means (8) being disposed using the multilayer technique.

5. An apparatus as set forth in Claims 1 and 2, characterized in that said scintillator (6) is coated on the optical fibers on entry (5b) of said screen (5) by depositing layers of crystals with a constant granulometry of 3-4 μ, the deposit having an overall thickness inferior to 100 μ in order to avoid the phenomena of optical diffusion, said deposit having an emission wavelength ranging from 500 to 800 nm (nanometers) corresponding to the optimal range of response for the charge-coupled device.

6. An apparatus as set forth in Claims 1, 2 and 3, characterized in that the metallic oxide particles loading the optical fibers of said screen (5) have an atomic mass superior by 50 % to the atomic mass of silicium constituting the stabilized glass in which said optical fibers are spun, in order to increase the degree of absorbtion of the screen upon the X-rays which have not been converted into visible radiation by the scintillator (6) and said metallic oxide particles have a tetravalent chemical structure and a melting point superior to 1 500 °C which allows spinning of reducing optical fibers containing said particles to diameters of a few microns.

7. An apparatus as set forth in claim 1 and 4, characterized in that the electric power supply of the microelectronic means (8) for monitoring the chip (4) of the charge-coupled device and for amplifying the output signal of said chip is synchronized with that of the X-ray generator (1) for the minimum period of time necessary to obtain one single image on the monitor of the display chain on which said image will be maintained for ulterior exploratory study.

8. An apparatus as set forth in claims 1, 2, 3, 5 and 6 taken together, characterized in that :

said screen (5), made of the reducing optical fibers, is shaped as a truncated pyramid whose top plane (5a) is parallel to the base (5b),

said scintillator (6) lies on said base (5b) of the pyramid and the sensible face of the chip (4) is stuck on the truncated top plane (5a) of said pyramid (5),

and a lead belt (9) whose plane is parallel to the base (5b) of the pyramid projects from the lateral faces of said pyramid, said lead belt being located at a height equal to the two-thirds of the total height of the pyramid starting from the base where is located the scintillator.

9. An apparatus as set forth in claims 1, 4 and 7, characterized in that some of the components (8a) of said microelectronic means (8) for monitoring said chip (4) and for amplifying the output signal of the chip, liable to raise the temperature around said chip are dissociated and displayed on lateral projections of said ceramic substrate (7), said projections (7a) being perpendicular to the remaining components stuck on the chip (4).

10. An apparatus as set forth in claims 1 to 9, characterized in that the outer faces of the constituents of the intraoral sensor (3) for the X-rays emerging from a radiated tooth (2) are embedded in a coating material (10) ensuring :

a selective passage for specific wavelengths, which permits the passage of the X-rays while preventing the passage of visible radiations external to the sensor (3),

a low coefficient of thermal conduction for limiting the heating of the chip (4) of the charge transfer device,

an electric insulation,

and a mechanical protection of the sensor (3) against impacts.

11. An apparatus as set forth in claims 1 to 10, characterized in that the dimensions of the various constituents of said intraoral sensor (3) for the X-ray beam emerging from a radiated tooth (2) are such as :

the dimensions of said intraoral sensor (3) allow easy introiduction and withdrawing in and from the mouth of said sensor,

the dimensions of the base (5b) of the truncated pyramid-shaped screen (5) on which the crystals of the scintillator (6) are deposited allow reception on entry of the screen of the full image of the X-ray beam emerging from at least one radiated tooth (2),

the dimensions of the truncated plane (5a) of said pyramid-shaped screen (5) on which is stuck the sensible face of said chip (4) of said charge-coupled device allow to obtain on issue of the screen (5) a dental image whose size is reduced enough to allow it to be registered by said sensible face of the chip (4),

and the height of the truncated pyramid-shaped screen (5) corresponds to the length of the optical fibers loaded with metallic oxide particles, and allows sufficient absorption of the energy of the X-rays which have not been converted into visible radiation by the scintillator (6), thus ensuring protection of the sensitive face of said chip.

12. An apparatus as set forth in claim 10, characterized in that said coating material (9) for the outer faces of the various constituents of said intraoral sensor (3) is black polyurethane resin.

13. An apparatus as set forth in claim 11, characterized in that the entry of the intraoral sensor (3) is a 30 mm high (h) and 20 mm wide (l) rectangle which corresponds to the base of said truncated pyramid and the total thickness (e) of said intraoral sensor is limited to 17 mm, which corresponds to the sum of the thickness of said scintillator (6), screen (5), chip (4), microelectronic means (8), and coating material.

14. An apparatus as set forth in claims 1 to 13, characterized in that said intraoral sensor (3) is positioned in the mouth by means of a movable and jointed arm.

15. An apparatus as set forth in claims 1 to 14, characterized in that said intraoral sensor (3) is placed in a throw-away aseptic pack.

**Patentansprüche**

1. Gerät zur Darstellung eines Dentalröntgenbildes auf einem Monitor einer Sichtbarmachungskette, mit

einer extrabukkalen Röntgenquelle,

einem intrabukkalen Sensor (3) für die Röntgenstrahlen, welche den bestrahlten Zahn (2) durchlaufen haben, wobei dieser Sensor (3) koaxial zu dem Röntgenstrahlbündel angeordnet ist, der von dem Zahn (2) ausgeht,

und einer elektronischen Bearbeitungseinheit, die extrabukkal angeordnet und mit dem Sensor (3) verbunden ist, wobei dieser Sensor (3) aus der Assoziierung einer Ladungsübertragungsvorrichtung (CCD) (4) mit einem Schirm (5) besteht, welcher zwischen dieser Ladungsübertragungsvorrichtung (CCD) (4) und dem bestrahlten Zahn (2) angeordnet und an seinem Eingang mit einem Szintillator (6) versehen ist, der die Röntgenstrahlen, die den Zahn (2) durchlaufen haben, in sichtbares Licht umwandelt, wobei der Schirm (5) aus einem durchscheinenden Material besteht, durch den die sichtbaren Strahlen durchtreten können, und mit Teilchen versehen ist, die undurchlässig sind für Röntgenstrahlen, welche den Szintillator (6) durchlaufen haben, von diesem jedoch nicht in sichtbares Licht umgewandelt wurden, dadurch gekennzeichnet,

daß einerseits in Übertragungsrichtung des Strahlungsverlaufes des Dentalbildes (Szintillator (6), Schirm (5), Ladungsübertragungsvorrichtung (CCD) (4)) die Durchmesser der Bauteile von gegenüberstehenden Bauteilpaaren einen zunehmenden Wert haben, nämlich die Durchmesser des ersten Paares von einander gegenüberstehenden Bauteilen, bestehend aus den Kristallen des Szintillators (6) und den Eintrittsflächen der optischen Fasern am Eingang des Schirmes (5), und die Durchmesser des zweiten Paares von einander gegenüberstehenden Bauteilen, bestehend aus den Ausgangsflächen der optischen Fasern am Ausgang des Schirmes (5) und den lichtempfindlichen Elementarbereichen der Ladungsübertragungsvorrichtung (4), um das Auflösungsvermögen der lichtempfindlichen Elementarbereiche der Ladungsübertragungsvorrichtung (CCD) (4) durch Vermindern des Flimmerns zu verbessern,

daß andererseits der Schirm (5) aus optischen Reduzierfasern gebildet ist, welche mit Metalloxidpartikeln versehen sind, um die Energie von Röntgenstrahlen zu absorbieren, welche vom Szintillator (6) nicht umgewandelt wurden,

und daß schließlich der intrabukkale Sensor (3) eine mikroelektronische Einrichtung (8) aufweist, um die Ladungsübertragungsvorrichtung (CCD) (4) zu führen und deren Ausgangssignal zu verstärken.

2. Gerät nach Anspruch 1, dadurch gekennzeichnet, daß die Kristalle des Szintillators (6) Abmessungen in der Größenordnung von 3 bis 4 Mikron, die optischen Fasern am Eingang des Schirmes (5) Abmessungen in der Größenordnung von 9 bis 14 Mikron und am Ausgang des Schirmes in der Größenordnung von 4,5 bis 7 Mikron, und die lichtempfindlichen Elementarbereiche der Ladungsübertragungsvorrichtung (CCD) Abmessungen in der Größenordnung von 24 bis 34 Mikron haben.

3. Gerät nach Anspruch 1, dadurch gekennzeichnet, daß die optischen Fasern aus einem stabilisierten Glas, mit geringem Bräunungseffekt, bestehen.

4. Gerät nach Anspruch 1, dadurch gekennzeichnet, daß der Chip (4), welcher den funktionierenden Teil der Ladungsübertragungsvorrichtung (CCD) bildet, aus seinem Gehäuse entnommen ist und daß seine gegenüber sichtbaren Strahlen empfindliche Fläche auf die optischen Fasern am Ausgang (5a) des Schirmes (5) und seine entgegengesetzte Fläche auf einen auch die Mikroelektronik (8) tragenden Keramikträger (7) aufgeklebt ist, wobei diese Mikroelektronik (8) den Chip leitet, dessen Ausgangssignal verstärkt und gemäß der Vielschichtentechnik aufgebaut ist.

5. Gerät nach den Ansprüchen 1 und 2, dadurch gekennzeichnet, daß der Szintillator (6) auf die optischen Fasern am Eingang (5b) des Schirmes (5) durch Aufbringen von Kristallschichten konstanter Granulometrie von 3 bis 4 Mikron aufgebracht wird, wobei die Gesamtdicke der Auflage unter 100 Mikron liegt, um hierbei die Lichtstreuung im Innern des Szintillators zu vermeiden, und wobei die Wellenlänge der Emission der Kristalle zwischen 500 nm und 800 nm (Namometern) liegt, entsprechend dem optimalen Ansprechbereich der Ladungsübertragungsvorrichtung (CCD).

6. Gerät nach den Ansprüchen 1, 2 und 3, dadurch gekennzeichnet, daß die Metalloxidpartikeln, mit denen die optischen Fasern des Schirmes (5) versehen sind, einerseits eine um 50 % höhere Atommasse haben als das Silizium, welches das stabilisierte Glas bildet, aus dem die optischen Fasern gemacht sind, um das Absorptionsvermögen des Schirmes zu verbessern, der auf die vom Szintillator (6) nicht umgewandelten Röntgenstrahlen einwirkt, und andererseits eine vierwertige chemische Struktur und einen Schmelzpunkt oberhalb von 1 500 °C haben, um das Ziehen der optischen Reduzierfasern bis zu Durchmessern von einigen Mikron zu erleichtern.

7. Gerät nach den Ansprüchen 1 und 4, dadurch gekennzeichnet, daß die elektrische Stromzuführung zur Mikroelektronik (8), welche den Chip (4) der Ladungsübertragungsvorrichtung (CCD) steuert und das Ausgangssignal des Chips verstärkt, mit der Röntgenquelle für eine Zeitdauer synchronisiert ist, welche erforderlich ist, um ein einziges Bild auf dem Monitor der Sichtbarmachungskette zu erhalten, auf welchem dieses Bild für weitere Untersuchungen festgehalten wird.

8. Gerät nach der Gesamtheit der Ansprüche 1, 2, 3, 5 und 6, dadurch gekennzeichnet, daß

einerseits der Schirm (5), der aus der Gesamtheit der optischen Reduzierfasern besteht, die allgemeine Form einer Pyramide aufweist, die in einer Ebene (5a) parallel zur Basisebene (5b) abgeschnitten ist,

andererseits der Szintillator auf der Basisebene (5b) der Pyramide angebracht und die den empfindlichen Teil des Chips (4) aufweisende Fläche auf die abgeschnittene Ebene (5a) der Pyramide (5) aufgeklebt ist,

und schließlich ein Bleigürtel (9), dessen Ebene parallel zur Basisebene (5b) der Pyramide ist, um die Seitenflächen dieser Pyramide angeordnet ist, und zwar auf einer Höhe von zwei Dritteln der Gesamthöhe der Pyramide über der Basisebene, wo sich der Szintillator befindet.

9. Gerät nach den Ansprüchen 1, 4 und 7, dadurch gekennzeichnet, daß gewisse Bauteile (8a) der Mikroelektronik (8), welche der Führung (Steuerung) des Chips (4) und der Verstärkung seines Ausgangssignales dienen und eine Temperaturerhöhung nahe dem Chip bewirken könnten, auf seitlichen Verlängerungen angebracht sind, welche auf senkrecht zum Keramikträger (7) vorstehenden Verlängerungen (7a) angeordnet sind, der (7) die anderen elektronischen Bauteile trägt und am Chip (4) anliegt.

10. Gerät nach der Gesamtheit der Ansprüche 1 bis 9, dadurch gekennzeichnet, daß alle Außenflächen des intrabukkalen Sensors (3) des Röntgenstrahlbündels, welches vom bestrahlten Zahn (2) ausgeht, in einem Umhüllungsmaterial eingebettet sind, welches gleichzeitig

den selektiven Durchgang gewisser Wellenlängen bewirkt, wobei insbesondere Röntgenstrahlen durchgelassen und sichtbare Strahlen von außerhalb des Sensors (3) ferngehalten werden,

eine geringe thermische Leitfähigkeit besitzt, um die Erwärmung des Chips der Ladungsübertragungsvorrichtung (CCD) zu vermindern,

eine elektrische Isolierfähigkeit besitzt, und den Sensor (3) gegen Stöße schützt.

11. Gerät nach der Gesamtheit der Ansprüche 1 bis 10, dadurch gekennzeichnet, daß die Abmessungen der verschiedenen Bauteile des intrabukkalen Sensors (3) des vom bestrahlten Zahn (2) ausgehenden Röntgenstrahlbündels derart festgelegt sind, daß

die Abmessungen des gesamten intrabukkalen Sensors (3) es ermöglichen, diesen leicht in den Mund einzuführen und aus diesem zu entnehmen,

die Abmessungen der Basisebene (5b) der abgeschnittenen Pyramide des Schirmes (5), auf welcher (5b) die Kristalle des Szintillators (6) angeordnet sind, es gestatten, am Eingang des Schirmes das gesamte Röntgenstrahlbündel zu empfangen, welches von mindestens einem bestrahlten Zahn ausgeht,

die Abmessungen der abgeschnittenen Ebene (5a) der Pyramide des Schirmes (5), auf welche die empfindliche Seite des Chips (4) der Ladungsübertragungsvorrichtung (CCD) aufgeklebt ist, es gestatten, am Ausgang des Schirms (5) ein hinreichend reduziertes Dentalbild zu erhalten, damit dieses auf der empfindlichen Fläche des Chips (4) registriert werden kann,

und die Abmessungen der Höhe der abgeschnittenen Pyramide des Schirmes (5), entsprechend der Länge der optischen Fasern und genauer der Dicke des mit Metalloxiden versehenen Glases, eine hinreichende Absorption der Energie der vom Szintillator (6) nicht umgeformten Röntgenstrahlen gestatten, um die empfindliche Fläche des Chips (4) zu schützen.

12. Gerät nach Anspruch 10, dadurch gekennzeichnet, daß das Ummantelungsmaterial (9) der Außenflächen der verschiedenen Bestandteile des intrabukkalen Sensors (3) aus schwarzem Polyurethanharz besteht.

13. Gerät nach Anspruch 11, dadurch gekennzeichnet, daß die Abmessungen des gesamten intrabukkalen Sensors (3) durch die Abmessungen eines Rechteckes am Eingang bestimmt sind, welches der Basisebene der abgeschnittenen Pyramide entspricht, und auf 30 mm Höhe (h), 20 mm Breite (l) und die Abmessungen der Gesamtdicke (e) begrenzt sind, welche der Summe der Dicken des Szintillators (6), des Schirmes (5), des Chips (4), der Mikroelektronik (8) und des Ummantelungsmaterials entspricht und auf 17 mm begrenzt ist.

14. Gerät nach der Gesamtheit der Ansprüche 1 bis 13, dadurch gekennzeichnet, daß der intrabukkale Sensor (3) in der Mundhöhle mittels eines gelenkigen und abnehmbaren Armes gehalten wird.

15. Gerät nach der Gesamtheit der Ansprüche 1 bis 14, dadurch gekennzeichnet, daß der intrabukkale Sensor (3) in einer aseptischen, wegwerfbaren Hülle untergebracht ist.